# FASCICULE DE BREVET EUROPEEN

(11) **EP 0 466 901 B1**
(45) Date de publication et mention de la délivrance du brevet: **24.07.1996**
(21) Numéro de dépôt: 91904078.2
(22) Date de dépôt: 07.02.1991
(51) Int. Cl.: C12N 15/53, C12N 9/04, C12N 1/21, C12P 33/02, C12Q 1/68, C12Q 1/32

(54) **SEGMENTS D'ADN ET MICRO-ORGANISMES TRANSFORMES COMPRENANT LE GENE DE LA DELTA-1 -DESHYDROGENASE, ET LEURS APPLICATIONS**
DNS-SEGMENTE UND DAS DELTA-1 DES HYDROGENASEGEN ENTHALTENDE MIKROORGANISMEN UND IHRE VERWENDUNG
DNA SEGMENTS AND TRANSFORMED MICROORGANISMS COMPRISING THE GENE OF DELTA-1 -DEHYDROGENASE, AND USES THEREOF

(30) Priorité: 07.02.1990 FR 9001399
(43) Date de publication de la demande: 22.01.1992
(73) Titulaire: ROUSSEL UCLAF, F-93230 Romainville (FR)
(72) Inventeur: MICHEL-BRIAND, Yvon, F-25000 Besançon (FR); PLESIAT, Patrick, F-25201 Roche-Lez-Beaupré (FR)
(74) Mandataire: Vieillefosse, Jean-Claude
(86) Numéro de dépôt international: FR9100094
(87) Numéro de publication internationale: WO9112326

(56) Documents cités:
- US-A- 3 734 830
- The Journal of Biological Chemistry, volume 234, no. 8, aout 1959, (US), H.R. Levy et al.: "Bacterial oxidation of steroids", pages 2014-2021

## Description

La présente invention est relative au clonage du gène de la delta¹-déshydrogénase de Pseudomonas testosteroni, à la séquence codant pour ledit gène, ainsi qu'à des micro-organismes transformés par introduction dudit gène et à leur utilisation pour la production de delta¹-déshygrogénase et pour la synthèse et le dosage de substances stéroïdiennes.

La biotransformation des stéroïdes par des micro-organismes, ou par des enzymes issues de ceux-ci, dans le but de synthétiser des médicaments est utilisée couramment dans l'industrie pharmaceutique, du fait de la très grande spécificité et du faible coût de revient des réactions effectuées de la sorte.

Par exemple, la delta¹-déshydrogénase obtenue à partir de certaines souches d'Athrobacter est couramment utilisée dans la synthèse des stérols à usage thérapeutique, tels que, par exemple, la prednisone et la prednisolone, à partir de substrats tels que, par exemple, la cortisone ou l'hydrocortisone.

Pseudomonas testosteroni est une bactérie à gram-négatif, se trouvant dans le sol, et qui est capable de croître en utilisant les stérols comme seule source de carbone. Cet organisme dégrade complètement les stérols, et plusieurs enzymes sont impliquées dans ce métabolisme:
- la 3-(ou 17)béta-hydroxystéroïde déshydrogénase (EC 1.1.1.51) désignée également sous le nom de béta-enzyme,
- la 3alpha-hydroxystéroïde déshydrogénase (EC 1.1.1.50) désignée également sous le nom de alpha-enzyme,
- la 3-cétostéroïde delta⁴-delta⁵-isomérase (EC 5.3.3.1) désignée également sous le nom d'isomérase,
- la 3-cétostéroïde delta¹-déshydrogénase (EC 1.3.99.4) désignée également sous le nom de delta¹-deshydrogénase,
ainsi que deux autres déshydrogénases (EC 1.3.99.5 et EC 1.3.99.6).

La delta¹-déshydrogénase de Pseudomonas testosteroni présente, par rapport aux enzymes de même type actuellement utilisées, l'avantage d'une grande stabilité physico-chimique.

Des tentatives pour isoler et caractériser cette enzyme ont été réalisées [LEVY et TALALAY, Journal of biological Chemistry (1959), 234, 2014-2021]; toutefois, elles n'ont pas abouti à la purification complète de la protéine, dont la séquence demeure, jusqu'à présent inconnue.

En outre, en raison de la complexité du métabolisme des stérols chez Pseudomonas testosteroni et des différentes enzymes impliquées dans ledit métabolisme, il est impossible d'utiliser directement Pseudomonas testosteroni dans l'industrie pour des réactions de synthèses spécifiques. Les inventeurs se sont en conséquence fixé pour but d'isoler et de cloner le gène de la delta¹-déshydrogénase de Pseudomonas testosteroni, de facon à permettre, d'une part l'expression dudit gène dans un hôte approprié, tel qu'une bactérie à usage industriel, et d'autre part l'obtention de la delta¹-déshydrogénase sous forme purifiée.

La présente invention a donc pour objet des segments d'acide nucléique, caractérisés en ce qu'ils comprennent au moins une séquence nucléotidique choisie dans le groupe constitué par :
a) une séquence nucléotidique codant pour la delta¹-déshydrogénase de Pseudomonas testosteroni,
b) une séquence nucléotidique régulant l'expression du gène de la delta¹-déshydrogénase de Pseudomonas testosteroni,
c) les séquences nucléotidiques homologues ou complémentaires de tout ou partie de l'une ou l'autre des séquences définies en a) ou b).

Au sens de la présente invention, on entend par "séquence homologue", non seulement les séquences identiques à celles définies en a) ou b), ou à un segment de celles-ci, mais également celles ne différant que par la substitution, la délétion ou l'addition d'un certain nombre de nucléotides, à condition que les séquences ainsi modifiées soient fonctionnellement équivalentes à la séquence ou au segment considéré.

De même, on entend par "séquence complémentaire", non seulement les séquences strictement complémentaires des séquences définies en a) ou b) ou de ses segments, mais également des séquences modifiées comme indiqué précédemment, à condition qu'elles soient fonctionnellement équivalentes auxdites séquences strictement complémentaires.

De telles séquences fonctionnellement équivalentes sont par exemple :
- des séquences d'ADN pouvant s'hybrider spécifiquement avec le gène de la delta¹-déshydrogénase, utilisables pour l'obtention de sondes permettant la sélection de recombinants ayant intégré ledit gène, lors du clonage de celui-ci.
- des séquences d'ADN codant pour la delta¹-déshydrogénase de P. testosteroni, et dérivées de la séquence (I) qui est définie ci-après à la suite de la description proprement dite, par remplacement d'un ou plusieurs codons de ladite séquence (I) par d'autres codons dont le produit de traduction est identique.

Des segments d'ADN conformes a l'invention peuvent être obtenus soit par l'une quelconque des techniques utilisables en biologie moléculaire ou en génie génétique, soit par l'association de plusieurs de ces techniques.

Lesdites techniques comprennent, sans que ceci constitue une énumération limitative, les techniques d'extraction et de purification des acides nucléiques, les techniques de fragmentation de molécules d'acide nucléique par des enzymes de restriction ou par toute autre méthode appropriée, les techniques de synthèse ou d'hemisynthèse d'acides nucléiques, les techniques de mutagénèse ou de transformation de micro-organismes, les techniques de marquage ou d'hybridation de sondes, les techniques de recombinaison d'acides nucléiques, etc...

Selon un mode de réalisation préféré d'un segment d'ADN conforme à l'invention, il comprend au moins une séquence homologue ou complémentaire de la séquence (I) ( désignée dans la liste des séquences en annexe sous le n° SEQ IDNO:1), et qui est celle du fragment KpnI de 2,2 kb environ, de l'ADN de Pseudomonas testosteroni.

De façon particulièrement avantageuse, un segment d'ADN conforme à l'invention comprend une séquence codant pour la delta¹-déshydrogénase de P. testosteroni, laquelle séquence commence au nucléotide 248 et finit au nucléotide 1966 de la séquence (I).

La présente invention a également pour objet des vecteurs recombinants, caractérisés en ce qu'ils contiennent un segment d'acide nucléique conforme à l'invention, tel que défini ci-dessus.

On entend par vecteur recombinant, une séquence d'acide nucléique résultant de la recombinaison d'une séquence d'acide nucléique capable de s'autorépliquer, et d'une séquence d' acide nucléique que l'on souhaite cloner et amplifier. Lesdits vecteurs recombinants peuvent également être des vecteurs d'expression, portant des séquences nucléotidiques qui contrôlent la transcription et la traduction d'une séquence codante insérée en un site approprié dudit vecteur.

De façon avantageuse, lesdits vecteurs contiennent en outre des séquences assurant la régulation de l'expression de ce gène telles que, par exemple, des séquences permettant un contrôle de l'expression dudit gène en fonction de conditions du milieu ambiant, comme par exemple la température.

Selon un mode de réalisation préféré de la présente invention, un vecteur recombinant est constitué par le plasmide dénommé pTEK21, lequel plasmide résulte de l'insertion dans le plasmide pUC18 du segment d'ADN de 2,2 kb défini plus haut.

Selon un autre mode de réalisation préféré de la présente invention, un tel vecteur d'expression est constitué par le plasmide recombinant dénommé pLE689, lequel résulte de l'insertion dans le plasmide pNM185 de la séquence nucléotidique codant pour la delta¹-déshydrogénase de Pseudomonas testosteroni, associée à d'autres séquences nucléotidiques issues du génôme de Pseudomonas Testosteroni, lesquelles séquences jouent un rôle dans l'expression et/ou dans la régulation de l'expression du gène de la delta¹-déshydrogénase.

Le plasmide pNM185 est décrit dans la publication de MERMOD et al., [J. Bacteriol., 167 : 447-454 (1986)].

La présente invention a également pour objet des micro-organismes transformés par l'un des vecteurs recombinants définis plus haut.

Des clones de Escherichia Coli, de Pseudomonas putida, et de Pseudomonas aeruginosa, transformés par les vecteurs mentionnés ci-dessus, ont fait l'objet d'un dépôt auprès de la Collection Nationale de Cultures de Micro-organismes (CNCM), le 24 Janvier 1990 :
- le clone E. coli DH5alpha transformé par le plasmide pTEK21 porte le numéro de dépôt I-922;
- le clone E. coli RR1 transformé par le plasmide pLE689 porte le numéro de dépôt I-923;
- le clone P. putida KT2440 transformé par le plasmide pLE689 porte le numéro de dépôt I-924;
- le clone P. aeruginosa PAO1161 transformé par le plasmide pLE689 porte le numéro de dépôt I-925;

Les micro-organismes transformés par l'un quelconque des plasmides conformes à l'invention sont utilisables pour le clonage et l'amplification dudit plasmide, portant le gène de la delta¹-déshydrogénase de Pseudomonas testosteroni. En outre, des micro-organismes appropriés, transformés par un plasmide capable de se répliquer dans ceux-ci et permettant l'expression du gène de la delta¹-déshydrogénase, peuvent être utilises, de façon avantageuse, dans la biotransformation des stéroïdes, car l'activité delta¹-déshydrogénase exprimée par ces micro-organismes n'est pas contaminée par d'autres activitées de dégradation des stéroïdes.

La présente invention a également pour objet des sondes d'acide nucléique permettant la détection du gène de la delta¹-déshydrogénase, ou d'un gène apparenté, chez Pseudomonas testosteroni, ou chez d'autres micro-organismes portant un tel gène, lesquelles sondes, sont caractérisées en ce qu'elles contiennent au moins un des segments d'acide nucléique conformes à l'invention, tels qu'ils ont été définis précédemment, associé à au moins un moyen de détection approprié.

La présente invention a en outre pour objet une chaine polypeptidique comprenant une séquence d'acides aminés dérivée de la séquence nucléotidique codant pour la delta¹-déshydrogénase de Pseudomonas testosteroni.

La présente invention a en outre pour objet un procédé de préparation de la delta¹-déshydrogénase de Pseudomonas testosteroni, lequel procédé est caractérisé en ce qu'il comprend les étapes suivantes :
a) culture d'un micro-organisme contenant un vecteur permettant l'expression du gène de la delta¹-déshydrogénase de Pseudomonas testosteroni,
b) isolement, à partir de ladite culture, de l'enzyme produite.

La delta¹-déshydrogénase ainsi purifiée peut être avantageusement utilisée pour le dosage ou la biosynthèse des stéroïdes.

La présente invention sera mieux comprise à l'aide du complément de description qui va suivre, qui se réfère à des exemples de préparation de segments d'acide nucléique conformes à l'invention. Il doit être bien entendu, toutefois, que ces exemples sont donnés uniquement à titre d'illustration de l'objet de l'invention, dont ils ne constituent en aucune manière une limitation.

### I) PURIFICATION ET CLONAGE DE SEGMENTS PORTANT LA SEQUENCE CODANT POUR LA DELTA¹-DESHYDROGENASE DE PSEUDOMONAS TESTOSTERONI.

### EXEMPLE 1 : Préparation d'une sonde permettant la localisation de la séquence codant pour la delta¹-déshydrogénase de Pseudomonas testosteroni.

Cette sonde a été obtenue à partir de l'ADN de mutants dérivés de la souche de P. testosteroni désignée par le numéro ATCC 17410.

### a) Mutagénèse

La mutagénèse de la souche P. testosteroni ATCC 17410 est réalisée en utilisant le transposon Tn5 portant un gène de résistance à la kanamycine, inséré dans le vecteur suicide pSUP2021. Ce vecteur est transféré à partir de d'E. coli S17-1. Lors de la conjugaison entre E. coli S17-1 et P. testosteroni ATCC 17410, le transfert du vecteur pSUP2021 se produit avec une fréquence élevée.

La sélection des clones de P. testosteroni contenant le transposon Tn5 est effectuée sur un milieu de culture minimum (milieu M9) contenant 5mM de parahydroxybenzoate de sodium, et 50 microgrammes/ml de kanamycine.

5200 clones de la souche ATCC 17410, resistants à la kanamycine sont isolés. Parmi ces clones, 41 n'utilisant pas la testostérone comme seule source de carbone sont sélectionnés. L'un de ces 41 clones (clone 06) présente une très faible activité delta¹-déshydrogénase.

L'ADN total du clone 06 est extrait et hydrolysé par EcoRI. Les fragments EcoRI ainsi obtenus ont été insérés dans pUC19 selon la procédure de religation aléatoire par la T4 ligase. Le mélange de ligation ainsi obtenu sert à transformer des cellules compétentes d'E. coli DH5alpha [fournies par GIBCO BRL, 14, rue des Oziers - 95051 CERGY PONTOISE CEDEX]. Les clones bactériens contenant le transposon Tn5 sont sélectionnés par leur résistance à la kanamycine. L'un de ces clones, porteur d'un plasmide recombinant pUC19 contenant un insert EcoRI d'environ 14 kb est choisi.

Un fragment BamHI-EcoRI de 0,8 kb, adjacent à Tn5 est sélectionné pour être utilisé comme sonde. Ce fragment est sous cloné dans le plasmide pUC19 suivant la procédure suivante : hydrolyse de l'insert EcoRI de 14 kb par BamHI, séparation du fragment BamHI-EcoRI par électrophorèse en gel d'agarose, purification par électroélution du gel, puis marquage par le [³²P] dCTP par la méthode dite de nick translation.

### EXEMPLE 2 : Obtention d'un fragment de 8,3 kb portant le gène de la delta¹-déshydrogénase.

L'ADN total de Pseudomonas testosteroni est extrait par précipitation à l'éthanol après lyse de la bactérie par le SDS. Cet ADN est hydrolysé par l'enzyme de restriction SalI dans les conditions suivantes : incubation à 37°C pendant 2 heures de l'ADN avec l'enzyme à raison de 3 unités par microgrammes d'ADN.

Les fragments obtenus sont insérés dans le plasmide pUC19, par l'action de la T4 ligase.

Les plasmides recombinants obtenus sont utilisés pour transformer la souche bactérienne E. coli DH5alpha dans les conditions définies par GIBCO BRL, fournisseur des cellules compétentes. La banque ainsi obtenue est criblée par hybridation avec la sonde de 0,8 kb obtenue selon la procédure décrite à l'exemple 1.

5 clones contenant un fragment SalI de 8,3 kb sont ainsi obtenus.

### EXEMPLE 3 : Préparation du plasmide pTEK21.

On procède au sous clonage du fragment KpnI de 2,1 kb à partir du fragment SalI de 8,3 kb : le fragment de 8,3 kb est hydrolysé par l'enzyme KpnI. Le fragment de 2,1 kb libéré est séparé par électrophorèse en gel d'agarose et purifié. Il est ensuite religué dans un vecteur pUC18, préalablement linéarisé par KpnI.

La carte de restriction de l'insert de 2,1 kb est illustrée par la figure 1.

La présence du gène de la delta¹-dèshydrogénase sur ce fragment de 2,1 kb est mise en évidence par la détection des produits résultant de l'action de cette enzyme. Le protocole opératoire utilisé pour cette détection est détaillé plus loin (exemple 3).

### II) SEQUENCAGE DU GENE DE LA DELTA¹-DESHYDROGENASE DE PSEUDOMONAS TESTOSTERONI.

Le séquencage du fragment KpnI de 2,1 kb est effectué en utilisant la méthode de SANGER de marquage enzymatique aux di-désoxynucléotides en présences de désoxyribonucléotides marqués au [³⁵S].

### III) MISE EN EVIDENCE DE L'EXPRESSION DU GENE DE LA DELTA¹-DESHYDROGENASE DE PSEUDOMONAS TESTOSTERONI DANS DES CELLULES BACTERIENNES TRANSFORMEES.

La souche E. coli DH5alpha renfermant le plasmide recombinant pTEK21 est cultivée en bouillon nutritif à 30°C, en présence de substrat de la delta¹-déshydrogénase (1,75 mM de delta⁴-androstène-3,17-dione). Après 16 h d'incubation, les stéroïdes sont extraits par l'acétate d'éthyle et analysés par chromatographie en couche mince de silice, en utilisant un mélange solvant dichlorométhane/dioxanne. Le métabolite delta¹-déshydrogéné (delta^{1,4}-androstadiène-3,17-dione) apparaît aux rayons ultra-violets sous forme d'une tache caractéristique.

Ainsi que celà ressort de ce qui précède, l'invention ne se limite nullement à ceux de ses modes de mise en oeuvre, de réalisation et d'application qui viennent d'être décrits de façon plus explicite; elle en embrasse au contraire toutes les variantes qui peuvent venir à l'esprit du technicien en la matière, sans s'écarter du cadre, ni de la portée, de la présente invention.

(I)
GGTACCTTCAAGAACAATCCCATCGAGCGCATCTTCCGCGACATCCATCAGG 52
GACGTACCCACATTGCGAACAATACGGATGCCTATGTGCGCGCCTATGGCTC 104
GCATGTGCTGGGATTCCCAACCAGGAACCTTTTGTCTGATTGAATTCATGCA 156
GCAAGCTGCGGCAGCGCTCCCATAACGGAGGCTGCCGCCAGCTGTATTTGCG 208
1
Met Ala Glu
ACAGTCCATCCATAAAACAAAGATTGACGGAGACAAGGT ATG GCA GAA 256
10
Gln Glu Tyr Asp Leu Ile Val Val Gly Ser Gly Ala Gly
CAA GAA TAT GAC CTG ATC GTC GTG GGT TCG GGG GCA GGT 295
20
Ala Cys Trp Ala Pro Ile Arg Ala Gln Glu Gln Gly Leu
GCC TGT TGG GCG CCA ATT CGC GCG CAG GAG CAG GGC CTC 334
30 40
Lys Thr Leu Val Val Glu Lys Thr Glu Leu Phe Gly Gly
AAG ACT CTG GTG GTG GAG AAG ACC GAG CTG TTC GGT GGC 373
50
Thr Ser Ala Leu Ser Gly Gly Gly Ile Trp Ile Pro Leu
ACT TCG GCT CTG TCG GGG GGT GGT ATC TGG ATT CCG CTC 412
60
Asn Tyr Asp Gln Lys Thr Ala Gly Ile Lys Asp Asp Leu
AAT TAC GAC CAG AAG ACC GCT GGC ATC AAA GAC GAT CTG 451
70 80
Glu Thr Ala Phe Gly Tyr Met Lys Arg Cys Val Arg Gly
GAA ACC GCA TTT GGC TAT ATG AAG CGC TGT GTG CGC GGC 490
90
Met Ala Thr Asp Asp Arg Val Leu Ala Tyr Val Glu Thr
ATG GCA ACC GAC GAC CGC GTG CTG GCC TAT GTG GAA ACC 529
100
Ala Ser Lys Met Ala Glu Tyr Leu Arg Gln Ile Gly Ile
GCG AGC AAG ATG GCC GAG TAC CTG CGC CAG ATC GGC ATC 568
110 120
Pro Tyr Arg Ala Met Ala Lys Tyr Ala Asp Tyr Tyr Pro
CCT TAT CGC GCC ATG GCC AAG TAT GCG GAC TAC TAC CCC 607
130
His Ile Glu Gly Ser Arg Pro Gly Gly Arg Thr Met Asp
CAT ATC GAA GGC TCC AGG CCC GGT GGC CGC ACC ATG GAC 646

140
Pro Val Asp Phe Asn Ala Ala Arg Leu Arg Val Thr Ala
CCG GTG GAC TTC AAT GCC GCC AGG CTG CGC GTG ACG GCA 685
150
Leu Glu Thr Met Arg Pro Gly Pro Pro Gly Asn Gln Leu
CTG GAA ACC ATG CGC CCC GGC CCT CCC GGC AAC CAG TTG 724
160 170
Phe Gly Arg Met Ser Ile Ser Ala Phe Glu Ala His Ser
TTC GGC CGC ATG AGC ATC AGT GCC TTC GAG GCG CAT TCC 763
180
Met Leu Ser Arg Glu Leu Lys Ser Arg Phe Thr Ile Leu
ATG CTC TCG CGC GAG CTC AAG TCG CGC TTC ACC ATC CTG 802
190
Gly Ile Met Leu Lys Tyr Phe Leu Asp Tyr Pro Trp Arg
GGC ATC ATG CTC AAG TAT TTT CTG GAC TAC CCC TGG CGC 841
200 210
Asn Lys Thr Arg Arg Asp Arg Arg Met Thr Gly Gly Gln
AAC AAG ACC AGG CGC GAT CGT CGC ATG ACG GGC GGC CAG 880
220
Ala Leu Val Ala Gly Leu Leu Thr Ala Ala Asn Lys Ala
GCG CTG GTG GCA GGC CTG CTG ACT GCT GCC AAC AAG GCC 919
230
Arg Val Glu Met Trp Cys Asn Ser Pro Leu Lys Glu Leu
CGC GTC GAG ATG TGG TGC AAC TCT CCG CTC AAG GAG CTG 958
240 250
Val Gln Asp Ala Ser Gly Arg Val Thr Gly Val Ile Val
GTG CAG GAT GCA TCG GGC CGC GTG ACG GGT GTC ATT GTT 997
260
Glu Arg Asn Gly Gln Arg Gln Gln Ile Asn Ala Arg Arg
GAG AGG AAT GGT CAG CGC CAG CAG ATC AAC GCC AGA CGT 1036
270
Gly Val Leu Leu Gly Ala Gly Gly Phe Glu Arg Asn Gln
GGC GTG TTG TTG GGT GCA GGC GGT TTT GAG CGC AAT CAG 1075
280
Glu Met Arg Asp Gln Tyr Leu Asn Lys Pro Thr Arg Leu
GAG ATG CGT GAC CAG TAT CTG AAC AAG CCC ACA AGG CTG 1114
290 300
Val Asp Gly Asn Pro Cys Gly Arg Gln Tyr Gly Asp Ala
GTG GAC GGC AAC CCC TGT GGG AGG CAA TAC GGT GAT GCT 1153

310
His Arg Ala Gly Gln Ala Trp Ala His Thr Gly Ala Asp
CAC CGG GCC GGT CAG GCG TGG GCG CAC ACT GGC GCT GAT 1192
320
Gly Leu Val Leu Gly Arg Ala His His Gly Cys Ser Gln
GGA CTG GTC CTG GGG CGT GCC CAC CAT GGA TGT TCC CAA 1231
330 340
Gly Ala Gly Leu Ser Arg His Phe Arg Gly Thr Leu Ala
GGA GCC GGC CTT TCG CGG CAT TTT CGT GGA ACG CTC GCT 1270
350
Ala Gly Val His Gly Gly Gln Arg Gln Gly Ala Ala Leu
GCC GGG GTG CAT GGT GGT CAA CGA CAA GGG GCA GCG CTT 1309
360
Pro Gln Arg Val Arg Pro Val Ser Gly Ile Pro Ala Ala
CCT CAA CGA GTC CGG CCC GTA TCC GGA ATT CCA GCA GCC 1348
370 380
Met Leu Ala Glu Asn Ala Lys Gly Asn Gly Gly Val Pro
ATG CTG GCC GAA AAT GCC AAG GGC AAT GGC GGT GTG CCT 1387
390
Ala Trp Ile Val Phe Asp Ala Ser Phe Arg Ala Gln Asp
GCA TGG ATT GTG TTC GAC GCC AGC TTC CGC GCG CAA AAC 1426
400
Pro Met Gly Pro Leu Met Pro Gly Ser Ala Val Pro Asp
CCC ATG GGG CCG CTG ATG CCA GGC TCG GCC GTG CCA GAC 1465
410
Ser Lys Val Arg Lys Ser Trp Leu Asn Asn Val Tyr Trp
AGC AAG GTG CGC AAG AGC TGG CTG AAC AAT GTC TAC TGG 1504
420 430
Lys Gly Arg Arg Trp Lys Ile Trp Arg Ala Asp Arg Arg
AAG GGC AGA CGC TGG AAG ATC TGG CGC GCA GAT CGG CGT 1543
440
Gly Arg Ala Gly Leu Gln Val Ser Ala Arg Arg Met Thr
GGA CGT GCT GGG CTG CAG GTC AGT GCG CGT CGC ATG ACC 1582
450
Glu Tyr Ala Arg Ala Gly Lys Asp Leu Asp Phe Asp Arg
GAA TAC GCC AGA GCT GGC AAG GAC CTG GAC TTT GAC CGG 1621

460                                                470
Gly Gly Asn Val Phe Asp Arg Tyr Tyr Gly Asp Pro Arg
GGC GGC AAT GTG TTT GAC CGC TAC TAC GGC GAT CCG CGT   1660
                                        480
Leu Lys Asn Pro Asn Leu Gly Pro Ile Glu Lys Gly Pro
CTC AAG AAT CCC AAC CTG GGT CCC ATC GAG AAA GGT CCG   1699
                        490
Phe Tyr Ala Met Arg Leu Trp Pro Gly Glu Ile Gly Thr
TTC TAC GCC ATG CGT CTG TGG CCC GGT GAG ATC GGC ACC   1738
        500                                     510
Lys Gly Gly Leu Leu Thr Asp Arg Glu Gly Arg Val Leu
AAG GGG GGG CTG CTG ACC GAC AGG GAA GGC CGT GTG CTC   1777
                                            520
Asp Thr Gln Gly Arg Ile Ile Glu Gly Leu Tyr Cys Val
GAC ACG CAA GGC AGG ATC ATC GAG GGG CTG TAT TGC GTG   1816
                            530
Gly Asn Asn Ser Ala Ser Val Met Ala Pro Ala Tyr Ala
GGC AAC AAC TCC GCC TCC GTC ATG GCG CCG GCC TAC GCC   1855
            540
Gly Ala Gly Ser Thr Leu Gly Pro Ala Met Thr Phe Ala
GGC GCT GGC TCC ACC CTG GGG CCG GCC ATG ACG TTT GCC   1894
550                                         560
Phe Arg Ala Val Ala Asp Met Val Gly Lys Pro Leu Pro
TTC CGC GCC GTG GCT GAC ATG GTA GGC AAA CCC TTG CCT   1933
                                570         573
Leu Glu Asn Pro His Leu Leu Gly Lys Thr Val
CTC GAG AAC CCG CAT CTG CTG GGC AAG ACG GTT TGACCAGG   1974
AGGCGCAGAGATGTCCAATGTCAAAAAGCATGTCAGCACCATCAATCCTGTG   2026
GGGGAAGTCCTGGATGTGGGGTCGGCCGATGAGGTTCAGTGGTCCGATGCCA   2078
GCGATGTGGTGGTGGTCGGCTGGGGAGGGGCAGGTGCCAGTGCAGCGATTGA   2130
GGCGCGCGAGCAGGGGGCAGAAGTTCTGGTGATCGAGCGCTTCAGCGGTGGC   2182
GGTGCCAGTGTGCTGTCGGGCGGTGTGGTCTATGCCGGTGGCGGTACC   2230

### LISTE DES SEQUENCES

- SEQ. ID NO : 1
- TYPE DE SEQUENCE : Nucléotide et sa protéine correspondante
- LONGUEUR DE LA SEQUENCE : 2230 paires de bases
- NOMBRE DE BRINS : Simple
- CONFIGURATION : Linéaire
- TYPE DE MOLECULE : ADN génomique
- ORIGINE : Pseudomonas testosteroni
- SOURCE EXPERIMENTALE : Immédiate
- PROPRIETE : Gène codant pour la delta-deshydrogénase de P. testosteroni.

## Revendications

1. Séquence d'acide nucléique, caractérisée en ce qu'elle comprend au moins une séquence nucléotidique choisie dans le groupe constitué par :
a) une séquence nucléotidique codant pour la delta¹-déshydrogénase de Pseudomonas testosteroni ayant la séquence (I) SEQ ID N°1, et
b) les séquences nucléotidiques homologues de la séquence définie en a) et codant pour une protéine ayant l'activité delta¹-déshydrogénase, et
c) les séquences nucléotidiques complémentaires des séquences définies en a) ou b).

2. Séquence d'acide nucléique selon la revendication 1, caractérisée en ce qu'elle a la séquence (I) suivante (SEQ ID N°1) :

(I)
GGTACCTTCAAGAACAATCCCATCGAGCGCATCTTCCGCGACATCCATCAGG 52
GACGTACCCACATTGCGAACAATACGGATGCCTATGTGCGCGCCTATGGCTC 104
GCATGTGCTGGGATTCCCAACCAGGAACCTTTTGTCTGATTGAATTCATGCA 156
GCAAGCTGCGGCAGCGCTCCCATAACGGAGGCTGCCGCCAGCTGTATTTGCG 208
1
Met Ala Glu
ACAGTCCATCCATAAAACAAAGATTGACGGAGACAAGGT ATG GCA GAA 256
10
Gln Glu Tyr Asp Leu Ile Val Val Gly Ser Gly Ala Gly
CAA GAA TAT GAC CTG ATC GTC GTG GGT TCG GGG GCA GGT 295
20
Ala Cys Trp Ala Pro Ile Arg Ala Gln Glu Gln Gly Leu
GCC TGT TGG GCG CCA ATT CGC GCG CAG GAG CAG GGC CTC 334
30 40
Lys Thr Leu Val Val Glu Lys Thr Glu Leu Phe Gly Gly
AAG ACT CTG GTG GTG GAG AAG ACC GAG CTG TTC GGT GGC 373
50
Thr Ser Ala Leu Ser Gly Gly Gly Ile Trp Ile Pro Leu
ACT TCG GCT CTG TCG GGG GGT GGT ATC TGG ATT CCG CTC 412
60
Asn Tyr Asp Gln Lys Thr Ala Gly Ile Lys Asp Asp Leu
AAT TAC GAC CAG AAG ACC GCT GGC ATC AAA GAC GAT CTG 451
70 80
Glu Thr Ala Phe Gly Tyr Met Lys Arg Cys Val Arg Gly
GAA ACC GCA TTT GGC TAT ATG AAG CGC TGT GTG CGC GGC 490
90
Met Ala Thr Asp Asp Arg Val Leu Ala Tyr Val Glu Thr
ATG GCA ACC GAC GAC CGC GTG CTG GCC TAT GTG GAA ACC 529
100
Ala Ser Lys Met Ala Glu Tyr Leu Arg Gln Ile Gly Ile
GCG AGC AAG ATG GCC GAG TAC CTG CGC CAG ATC GGC ATC 568
110 120
Pro Tyr Arg Ala Met Ala Lys Tyr Ala Asp Tyr Tyr Pro
CCT TAT CGC GCC ATG GCC AAG TAT GCG GAC TAC TAC CCC 607
130
His Ile Glu Gly Ser Arg Pro Gly Gly Arg Thr Met Asp
CAT ATC GAA GGC TCC AGG CCC GGT GGC CGC ACC ATG GAC 646

140

Pro Val Asp Phe Asn Ala Ala Arg Leu Arg Val Thr Ala
CCG GTG GAC TTC AAT GCC GCC AGG CTG CGC GTG ACG GCA 685
150
Leu Glu Thr Met Arg Pro Gly Pro Pro Gly Asn Gln Leu
CTG GAA ACC ATG CGC CCC GGC CCT CCC GGC AAC CAG TTG 724
160 170
Phe Gly Arg Met Ser Ile Ser Ala Phe Glu Ala His Ser
TTC GGC CGC ATG AGC ATC AGT GCC TTC GAG GCG CAT TCC 763
180
Met Leu Ser Arg Glu Leu Lys Ser Arg Phe Thr Ile Leu
ATG CTC TCG CGC GAG CTC AAG TCG CGC TTC ACC ATC CTG 302
190
Gly Ile Met Leu Lys Tyr Phe Leu Asp Tyr Pro Trp Arg
GGC ATC ATG CTC AAG TAT TTT CTG GAC TAC CCC TGG CGC 341
200 210
Asn Lys Thr Arg Arg Asp Arg Arg Met Thr Gly Gly Gln
AAC AAG ACC AGG CGC GAT CGT CGC ATG ACG GGC GGC CAG 880
220
Ala Leu Val Ala Gly Leu Leu Thr Ala Ala Asn Lys Ala
GCG CTG GTG GCA GGC CTG CTG ACT GCT GCC AAC AAG GCC 919
230
Arg Val Glu Met Trp Cys Asn Ser Pro Leu Lys Glu Leu
CGC GTC GAG ATG TGG TGC AAC TCT CCG CTC AAG GAG CTG 958
240 250
Val Gln Asp Ala Ser Gly Arg Val Thr Gly Val Ile Val
GTG CAG GAT GCA TCG GGC CGC GTG ACG GGT GTC ATT GTT 997
260
Glu Arg Asn Gly Gln Arg Gln Gln Ile Asn Ala Arg Arg
GAG AGG AAT GGT CAG CGC CAG CAG ATC AAC GCC AGA CGT 1036
270
Gly Val Leu Leu Gly Ala Gly Gly Phe Glu Arg Asn Gln
GGC GTG TTG TTG GGT GCA GGC GGT TTT GAG CGC AAT CAG 1075
280
Glu Met Arg Asp Gln Tyr Leu Asn Lys Pro Thr Arg Leu
GAG ATG CGT GAC CAG TAT CTG AAC AAG CCC ACA 'AGG CTG 1114
290 300
Val Asp Gly Asn Pro Cys Gly Arg Gln Tyr Gly Asp Ala
GTG GAC GGC AAC CCC TGT GGG AGG CAA TAC GGT GAT GCT 1153

310
His Arg Ala Gly Gln Ala Trp Ala His Thr Gly Ala Asp
CAC CGG GCC GGT CAG GCG TGG GCG CAC ACT GGC GCT GAT 1192
320
Gly Leu Val Leu Gly Arg Ala His His Gly Cys Ser Gln
GGA CTG GTC CTG GGG CGT GCC CAC CAT GGA TGT TCC CAA 1231
330 340
Gly Ala Gly Leu Ser Arg His Phe Arg Gly Thr Leu Ala
GGA GCC GGC CTT TCG CGG CAT TTT CGT GGA ACG CTC GCT 1270
350
Ala Gly Val His Gly Gly Gln Arg Gln Gly Ala Ala Leu
GCC GGG GTG CAT GGT GGT CAA CGA CAA GGG GCA GCG CTT 1309
360
Pro Gln Arg Val Arg Pro Val Ser Gly Ile Pro Ala Ala
CCT CAA CGA GTC CGG CCC GTA TCC GGA ATT CCA GCA GCC 1348
370 380
Met Leu Ala Glu Asn Ala Lys Gly Asn Gly Gly Val Pro
ATG CTG GCC GAA AAT GCC AAG GGC AAT GGC GGT GTG CCT 1387
390
Ala Trp Ile Val Phe Asp Ala Ser Phe Arg Ala Gln Asp
GCA TGG ATT GTG TTC GAC GCC AGC TTC CGC GCG CAA AAC 1426
400
Pro Met Gly Pro Leu Met Pro Gly Ser Ala Val Pro Asp
CCC ATG GGG CCG CTG ATG CCA GGC TCG GCC GTG CCA GAC 1465
410
Ser Lys Val Arg Lys Ser Trp Leu Asn Asn Val Tyr Trp
AGC AAG GTG CGC AAG AGC TGG CTG AAC AAT GTC TAC TGG 1504
420 430
Lys Gly Arg Arg Trp Lys Ile Trp Arg Ala Asp Arg Arg
AAG GGC AGA CGC TGG AAG ATC TGG CGC GCA GAT CGG CGT 1543
440
Gly Arg Ala Gly Leu Gln Val Ser Ala Arg Arg Met Thr
GGA CGT GCT GGG CTG CAG GTC AGT GCG CGT CGC ATG ACC 1582
450
Glu Tyr Ala Arg Ala Gly Lys Asp Leu Asp Phe Asp Arg
GAA TAC GCC AGA GCT GGC AAG GAC CTG GAC TTT GAC CGG 1621

460                                                    470
Gly Gly Asn Val Phe Asp Arg Tyr Tyr Gly Asp Pro Arg
GGC GGC AAT GTG TTT GAC CGC TAC TAC GGC GAT CCG CGT   1660
                                        480
Leu Lys Asn Pro Asn Leu Gly Pro Ile Glu Lys Gly Pro
CTC AAG AAT CCC AAC CTG GGT CCC ATC GAG AAA GGT CCG   1699
                          490
Phe Tyr Ala Met Arg Leu Trp Pro Gly Glu Ile Gly Thr
TTC TAC GCC ATG CGT CTG TGG CCC GGT GAG ATC GGC ACC   1738
        500                                         510
Lys Gly Gly Leu Leu Thr Asp Arg Glu Gly Arg Val Leu
AAG GGG GGG CTG CTG ACC GAC AGG GAA GGC CGT GTG CTC   1777
                                            520
Asp Thr Gln Gly Arg Ile Ile Glu Gly Leu Tyr Cys Val
GAC ACG CAA GGC AGG ATC ATC GAG GGG CTG TAT TGC GTG   1816
                              530
Gly Asn Asn Ser Ala Ser Val Met Ala Pro Ala Tyr Ala
GGC AAC AAC TCC GCC TCC GTC ATG GCG CCG GCC TAC GCC   1855
                540
Gly Ala Gly Ser Thr Leu Gly Pro Ala Met Thr Phe Ala
GGC GCT GGC TCC ACC CTG GGG CCG GCC ATG ACG TTT GCC   1894
550                                             560
Phe Arg Ala Val Ala Asp Met Val Gly Lys Pro Leu Pro
TTC CGC GCC GTG GCT GAC ATG GTA GGC AAA CCC TTG CCT   1933
                                    570           573
Leu Glu Asn Pro His Leu Leu Gly Lys Thr Val
CTC GAG AAC CCG CAT CTG CTG GGC AAG ACG GTT TGACCAGG   1974
AGGCGCAGAGATGTCCAATGTCAAAAAGCATGTCAGCACCATCAATCCTGTG   2026
GGGGAAGTCCTGGATGTGGGGTCGGCCGATGAGGTTCAGTGGTCCGATGCCA   2078
GCGATGTGGTGGTGGTCGGCTGGGGAGGGGCAGGTGCCAGTGCAGCGATTGA   2130
GGCGCGCGAGCAGGGGGCAGAAGTTCTGGTGATCGAGCGCTTCAGCGGTGGC   2182
GGTGCCAGTGTGCTGTCGGGCGGTGTGGTCTATGCCGGTGGCGGTACC   2230

3. Séquence d'acide nucléique selon l'une quelconque des revendications 1 ou 2, caractérisée en ce que la séquence codant pour la deltal-déshydrogénase de P. testosteroni commence au nucléotide 248 et finit au nucléotide 1966 de la séquence (I) SEQ ID N°1.

4. Vecteurs recombinants, caractérisés en ce qu'ils comprennent une séquence d'acide nucléique selon l'une quelconque des revendications 1 à 3.

5. Vecteur recombinant selon la revendication 4, caractérisé en ce qu'il est constitué par la plasmide dénommé pTEK21, lequel plasmide résulte de l'insertion dans le plasmide pUC18 d'une séquence d'ADN ayant la séquence SEQ ID N°1.

6. Vecteur recombinant selon la revendication 4, caractérisé en ce qu'il est constitué par le plasmide recombinant dénommé pLE689, lequel plasmide résulte de l'insertion dans la plasmide pNM185 de la séquence nucléotidique codant pour la delta¹ deshydrogénase de P. testosteroni associée à d'autres séquences nucléotidiques issues du génome de P. testosteroni.

7. Clones de micro-organismes transformés, caractérisés en ce qu'ils contiennent au moins un vecteur recombinant selon l'une quelconque des revendications 4 à 6.

8. Clone selon la revendication 7, déposé à la Collection National de Cultures de Micro-organismes (CNCM) sous le N°I-922, et constitué par des cellules bactériennes de la souche E. coli DH5alpha, contenant le plasmide pTEK21.

9. Clone selon la revendication 7, déposé à la Collection Nationale de Cultures de Micro-organismes (CNCM) sous le N°I-923, et constitué par des cellules bactériennes de la souche E. coli RR1, contenant le plasmide pLE689.

10. Clone selon la revendication 7, déposé à la Collection Nationale de Cultures de Micro-organismes (CNCM) sous le N°I-924, et constitué par des cellules bactériennes de la souche P. putida KT2440, contenant le plasmide pLE689.

11. Clone selon la revendication 7, déposé à la Collection Nationale de Cultures de Micro-organismes (CNCM) sous le N°I-925, et constitué par des cellules bactériennes de la souche P. aeruginosa PA01161, contenant le plasmide pLE689.

12. Application des micro-organismes transformés par un vecteur recombinant selon la revendication 4, à la biotransformation des stéroïdes.

13. Application des micro-organismes transformés par un vecteur recombinant selon la revendication 4, à la production de delta¹-déshydrogénase.

14. Sondes de détection caractérisées en ce qu'elles contiennent au moins une séquence d'acide nucléique selon l'une quelconque des revendications 1 ou 2, associé à au moins un moyen de détection approprié.

15. Chaîne polypeptidique comprenant une séquence d'acides aminés résultant de la traduction de la séquence d'ADN selon la revendication 3.

16. Procédé de préparation de la deltal-déshydrogénase de Pseudomonas testosteroni, caractérisé en ce qu'il comprend les étages suivantes :
a) culture d'un micro-organisme contenant un vecteur selon la revendication 4 permettant l'expression du gène de la delta¹-déshydrogénase de Pseudomonas testosteroni,
b) isolement, à partir de la culture, de la delta¹-déshydrogénase produite par ledit micro-organisme.

17. Application de la delta¹-déshydrogénase de Pseudomonas testosteroni, purifiée par le procédé selon la revendication 16, à la synthèse des stéroïdes.

18. Application de la delta¹-déshydrogénase de Pseudomonas testosteroni, purifiée par le procédé selon la revendication 16, au dosage des stéroïdes.

## Patentansprüche

1. Nukleinsäuresequenz, dadurch **gekennzeichnet**, daß sie mindestens eine Nukleotidsequenz, ausgewählt aus der Gruppe, bestehend aus
a) einer Nukleotidsequenz, die δ¹-Dehydrogenase von Pseudomonas testosteroni mit der Sequenz (I) SEQ ID Nr. 1 codiert, und
b) Nukleotidsequenzen, die der in a) definierten Sequenz homolog sind und ein Protein mit δ¹-Dehydrogenase-Aktivität codieren, und
c) den in a) und b) definierten Nukleotidsequenzen komplementären Sequenzen, umfaßt.

2. Nukleinsäuresequenz nach Anspruch 1, dadurch **gekennzeichnet**, daß sie die nachstehende Sequenz (I) (SEQ ID Nr.1) besitzt:

(I)
GGTACCTTCAAGAACAATCCCATCGAGCGCATCTTCCGCGACATCCATCAGG 52
GACGTACCCACATTGCGAACAATACGGATGCCTATGTGCGCGCCTATGGCTC 104
GCATGTGCTGGGATTCCCAACCAGGAACCTTTTGTCTGATTGAATTCATGCA 156
GCAAGCTGCGGCAGCGCTCCCATAACGGAGGCTGCCGCCAGCTGTATTTGCG 208
1
Met Ala Glu
ACAGTCCATCCATAAAACAAAGATTGACGGAGACAAGGT ATG GCA GAA 256
10
Gln Glu Tyr Asp Leu Ile Val Val Gly Ser Gly Ala Gly
CAA GAA TAT GAC CTG ATC GTC GTG GGT TCG GGG GCA GGT 295
20
Ala Cys Trp Ala Pro Ile Arg Ala Gln Glu Gln Gly Leu
GCC TGT TGG GCG CCA ATT CGC GCG CAG GAG CAG GGC CTC 334
30 40
Lys Thr Leu Val Val Glu Lys Thr Glu Leu Phe Gly Gly
AAG ACT CTG GTG GTG GAG AAG ACC GAG CTG TTC GGT GGC 373
50
Thr Ser Ala Leu Ser Gly Gly Gly Ile Trp Ile Pro Leu
ACT TCG GCT CTG TCG GGG GGT GGT ATC TGG ATT CCG CTC 412
60
Asn Tyr Asp Gln Lys Thr Ala Gly Ile Lys Asp Asp Leu
AAT TAC GAC CAG AAG ACC GCT GGC ATC AAA GAC GAT CTG 451
70 80
Glu Thr Ala Phe Gly Tyr Met Lys Arg Cys Val Arg Gly
GAA ACC GCA TTT GGC TAT ATG AAG CGC TGT GTG CGC GGC 490
90
Met Ala Thr Asp Asp Arg Val Leu Ala Tyr Val Glu Thr
ATG GCA ACC GAC GAC CGC GTG CTG GCC TAT GTG GAA ACC 529
100
Ala Ser Lys Met Ala Glu Tyr Leu Arg Gln Ile Gly Ile
GCG AGC AAG ATG GCC GAG TAC CTG CGC CAG ATC GGC ATC 568
110 120
Pro Tyr Arg Ala Met Ala Lys Tyr Ala Asp Tyr Tyr Pro
CCT TAT CGC GCC ATG GCC AAG TAT GCG GAC TAC TAC CCC 607
130
His Ile Glu Gly Ser Arg Pro Gly Gly Arg Thr Met Asp
CAT ATC GAA GGC TCC AGG CCC GGT GGC CGC ACC ATG GAC 646

140
Pro Val Asp Phe Asn Ala Ala Arg Leu Arg Val Thr Ala
CCG GTG GAC TTC AAT GCC GCC AGG CTG CGC GTG ACG GCA 685
150
Leu Glu Thr Met Arg Pro Gly Pro Pro Gly Asn Gln Leu
CTG GAA ACC ATG CGC CCC GGC CCT CCC GGC AAC CAG TTG 724
160 170
Phe Gly Arg Met Ser Ile Ser Ala Phe Glu Ala His Ser
TTC GGC CGC ATG AGC ATC AGT GCC TTC GAG GCG CAT TCC 763
180
Met Leu Ser Arg Glu Leu Lys Ser Arg Phe Thr Ile Leu
ATG CTC TCG CGC GAG CTC AAG TCG CGC TTC ACC ATC CTG 802
190
Gly Ile Met Leu Lys Tyr Phe Leu Asp Tyr Pro Trp Arg
GGC ATC ATG CTC AAG TAT TTT CTG GAC TAC CCC TGG CGC 841
200 210
Asn Lys Thr Arg Arg Asp Arg Arg Met Thr Gly Gly Gln
AAC AAG ACC AGG CGC GAT CGT CGC ATG ACG GGC GGC CAG 880
220
Ala Leu Val Ala Gly Leu Leu Thr Ala Ala Asn Lys Ala
GCG CTG GTG GCA GGC CTG CTG ACT GCT GCC AAC AAG GCC 919
230
Arg Val Glu Met Trp Cys Asn Ser Pro Leu Lys Glu Leu
CGC GTC GAG ATG TGG TGC AAC TCT CCG CTC AAG GAG CTG 958
240 250
Val Gln Asp Ala Ser Gly Arg Val Thr Gly Val Ile Val
GTG CAG GAT GCA TCG GGC CGC GTG ACG GGT GTC ATT GTT 997
260
Glu Arg Asn Gly Gln Arg Gln Gln Ile Asn Ala Arg Arg
GAG AGG AAT GGT CAG CGC CAG CAG ATC AAC GCC AGA CGT 1036
270
Gly Val Leu Leu Gly Ala Gly Gly Phe Glu Arg Asn Gln
GGC GTG TTG TTG GGT GCA GGC GGT TTT GAG CGC AAT CAG 1075
280
Glu Met Arg Asp Gln Tyr Leu Asn Lys Pro Thr Arg Leu
GAG ATG CGT GAC CAG TAT CTG AAC AAG CCC ACA AGG CTG 1114
290 300
Val Asp Gly Asn Pro Cys Gly Arg Gln Tyr Gly Asp Ala
GTG GAC GGC AAC CCC TGT GGG AGG CAA TAC GGT GAT GCT 1153

310
His Arg Ala Gly Gln Ala Trp Ala His Thr Gly Ala Asp
CAC CGG GCC GGT CAG GCG TGG GCG CAC ACT GGC GCT GAT 1192
320
Gly Leu Val Leu Gly Arg Ala His His Gly Cys Ser Gln
GGA CTG GTC CTG GGG CGT GCC CAC CAT GGA TGT TCC CAA 1231
330 340
Gly Ala Gly Leu Ser Arg His Phe Arg Gly Thr Leu Ala
GGA GCC GGC CTT TCG CGG CAT TTT CGT GGA ACG CTC GCT 1270
350
Ala Gly Val His Gly Gly Gln Arg Gln Gly Ala Ala Leu
GCC GGG GTG CAT GGT GGT CAA CGA CAA GGG GCA GCG CTT 1309
360
Pro Gln Arg Val Arg Pro Val Ser Gly Ile Pro Ala Ala
CCT CAA CGA GTC CGG CCC GTA TCC GGA ATT CCA GCA GCC 1348
370 380
Met Leu Ala Glu Asn Ala Lys Gly Asn Gly Gly Val Pro
ATG CTG GCC GAA AAT GCC AAG GGC AAT GGC GGT GTG CCT 1387
390
Ala Trp Ile Val Phe Asp Ala Ser Phe Arg Ala Gln Asp
GCA TGG ATT GTG TTC GAC GCC AGC TTC CGC GCG CAA AAC 1426
400
Pro Met Gly Pro Leu Met Pro Gly Ser Ala Val Pro Asp
CCC ATG GGG CCG CTG ATG CCA GGC TCG GCC GTG CCA GAC 1465
410
Ser Lys Val Arg Lys Ser Trp Leu Asn Asn Val Tyr Trp
AGC AAG GTG CGC AAG AGC TGG CTG AAC AAT GTC TAC TGG 1504
420 430
Lys Gly Arg Arg Trp Lys Ile Trp Arg Ala Asp Arg Arg
AAG GGC AGA CGC TGG AAG ATC TGG CGC GCA GAT CGG CGT 1543
440
Gly Arg Ala Gly Leu Gln Val Ser Ala Arg Arg Met Thr
GGA CGT GCT GGG CTG CAG GTC AGT GCG CGT CGC ATG ACC 1582
450
Glu Tyr Ala Arg Ala Gly Lys Asp Leu Asp Phe Asp Arg
GAA TAC GCC AGA GCT GGC AAG GAC CTG GAC TTT GAC CGG 1621

460                                                    470
Gly Gly Asn Val Phe Asp Arg Tyr Tyr Gly Asp Pro Arg
GGC GGC AAT GTG TTT GAC CGC TAC TAC GGC GAT CCG CGT    1660
                                        480
Leu Lys Asn Pro Asn Leu Gly Pro Ile Glu Lys Gly Pro
CTC AAG AAT CCC AAC CTG GGT CCC ATC GAG AAA GGT CCG    1699
                    490
Phe Tyr Ala Met Arg Leu Trp Pro Gly Glu Ile Gly Thr
TTC TAC GCC ATG CGT CTG TGG CCC GGT GAG ATC GGC ACC    1738
        500                                         510
Lys Gly Gly Leu Leu Thr Asp Arg Glu Gly Arg Val Leu
AAG GGG GGG CTG CTG ACC GAC AGG GAA GGC CGT GTG CTC    1777
                                            520
Asp Thr Gln Gly Arg Ile Ile Glu Gly Leu Tyr Cys Val
GAC ACG CAA GGC AGG ATC ATC GAG GGG CTG TAT TGC GTG    1816
                                530
Gly Asn Asn Ser Ala Ser Val Met Ala Pro Ala Tyr Ala
GGC AAC AAC TCC GCC TCC GTC ATG GCG CCG GCC TAC GCC    1855
            540
Gly Ala Gly Ser Thr Leu Gly Pro Ala Met Thr Phe Ala
GGC GCT GGC TCC ACC CTG GGG CCG GCC ATG ACG TTT GCC    1894
550                                             560
Phe Arg Ala Val Ala Asp Met Val Gly Lys Pro Leu Pro
TTC CGC GCC GTG GCT GAC ATG GTA GGC AAA CCC TTG CCT    1933
                                    570         573
Leu Glu Asn Pro His Leu Leu Gly Lys Thr Val
CTC GAG AAC CCG CAT CTG CTG GGC AAG ACG GTT TGACCAGG    1974
AGGCGCAGAGATGTCCAATGTCAAAAAGCATGTCAGCACCATCAATCCTGTG    2026
GGGGAAGTCCTGGATGTGGGGTCGGCCGATGAGGTTCAGTGGTCCGATGCCA    2078
GCGATGTGGTGGTGGTCGGCTGGGGAGGGGCAGGTGCCAGTGCAGCGATTGA    2130
GGCGCGCGAGCAGGGGGCAGAAGTTCTGGTGATCGAGCGCTTCAGCGGTGGC    2182
GGTGCCAGTGTGCTGTCGGGCGGTGTGGTCTATGCCGGTGGCGGTACC    2230

3. Nukleinsäuresequenz nach einem der Ansprüche 1 oder 2, dadurch **gekennzeichnet,** daß die die δ¹-Dehydrogenase von P. testosteroni codierende Sequenz bei Nukleotid 248 beginnt und bei Nukleotid 1966 der Sequenz (I) (SEQ ID Nr. 1) endet.

4. Rekombinante Vektoren, dadurch **gekennzeichnet,** daß sie eine Nukleinsäuresequenz nach einem der Ansprüche 1 bis 3 umfassen.

5. Rekombinanter Vektor nach Anspruch 4, dadurch **gekennzeichnet,** daß er aus dem Plasmid mit der Bezeichnung pTEK21 besteht, wobei das Plasmid das Ergebnis der Insertion einer DNA-Sequenz mit der Sequenz SEQ ID Nr. 1 in das Plasmid pUC18 ist.

6. Rekombinanter Vektor nach Anspruch 4, dadurch **gekennzeichnet,** daß er aus dem rekombinanten Plasmid mit der Bezeichnung pLE689 besteht, wobei das Plasmid das Ergebnis der Insertion der δ¹-Dehydrogenase von P. testosteroni codierenden Nukleotidsequenz in Verknüpfung mit anderen Nukleotidsequenzen aus dem Genom von P. testosteroni in das Plasmid pNM185 ist.

7. Clone von transformierten Mikroorganismen, dadurch **gekennzeichnet,** daß sie mindestens einen rekombinanten Vektor nach einem der Ansprüche 4 bis 6 enthalten.

8. Clon nach Anspruch 7, hinterlegt bei der Collection Nationale de Cultures de Microorganismes (CNCM) unter der Hinterlegungsnr. I-922 und bestehend aus Bakterienzellen des Stammes E. coli DH5α, die das Plasmid pTEK21 enthalten.

9. Clon nach Anspruch 7, hinterlegt bei der Collection Nationale de Cultures de Microorganismes (CNCM) unter der Hinterlegungsnr. I-923 und bestehend aus bakteriellen Zellen des Stammes E. coli RR1, die das Plasmid pLE689 enthalten.

10. Clon nach Anspruch 7, hinterlegt bei der Collection Nationale de Cultures de Microorganismes (CNCM) unter der Hinterlegungsnr. I-924 und bestehend aus Bakterienzellen des Stammes P. putida KT2440, die das Plasmid pLE689 enthalten.

11. Clon nach Anspruch 7, hinterlegt bei der Collection Nationale de Cultures de Microorganismes (CNCM) unter der Hinterlegungsnr. I-925 und bestehend aus Bakterienzellen des Stammes P. aeruginosa PA01161, die das Plasmid pLE689 enthalten.

12. Verwendung von mit einem rekombinanten Vektor nach Anspruch 4 transformierten Mikroorganismen zur Biotransformation Von Steroiden.

13. Verwendung von mit einem rekombinanten Vektor nach Anspruch 4 transformierten Mikroorganismen zur Produktion von δ¹-Dehydrogenase.

14. Detektionssonden, dadurch **gekennzeichnet,** daß sie mindestens eine Nukleinsäuresequenz nach einem der Ansprüche 1 oder 2, in Verknüpfung mit mindestens einem geeigneten Detektionsmittel, enthalten.

15. Polypeptidkette, umfassend eine Aminosäuresequenz als Ergebnis der Translation der DNA-Sequenz nach Anspruch 3.

16. Verfahren zur Produktion von δ¹-Dehydrogenase von Pseudomonas testosteroni, dadurch **gekennzeichnet**, daß es die folgenden Stufen umfaßt:
a) Züchtung eines Mikroorganismus, der einen Vektor nach Anspruch 4 enthält, der die Expression des Gens von δ¹-Dehydrogenase von Pseudomonas testosteroni erlaubt,
b) Isolieren der durch den Mikroorganismus produzierten δ¹-Dehydrogenase aus der Kultur.

17. Verwendung von nach dem Verfahren gemäß Anspruch 16 gereinigter δ¹-Dehydrogenase von Pseudomonas testosteroni zur Synthese von Steroiden.

18. Verwendung von nach dem Verfahren nach Anspruch 16 gereinigter δ¹-Dehydrogenase von Pseudomonas testosteroni zur Bestimmung von Steroiden.

## Claims

1. Nucleic acid sequence, characterized in that it contains at least one nucleotide sequence chosen from the group constituted by:
a) a nucleotide sequence coding for delta¹-dehydrogenase of Pseudomonas testosteroni having the sequence (I) SEQ ID No. 1, and
b) the nucleotide sequences homologous to the sequence defined in a) and coding for a protein having delta¹-dehydrogenase activity, and
c) the nucleotide sequences complementary to the sequences defined in a) or b).

2. Nucleic acid sequence according to claim 1, characterized in that it has the following sequence (I) (SEQ ID No. 1):

(I)
GGTACCTTCAAGAACAATCCCATCGAGCGCATCTTCCGCGACATCCATCAGG 52
GACGTACCCACATTGCGAACAATACGGATGCCTATGTGCGCGCCTATGGCTC 104
GCATGTGCTGGGATTCCCAACCAGGAACCTTTTGTCTGATTGAATTCATGCA 156
GCAAGCTGCGGCAGCGCTCCCATAACGGAGGCTGCCGCCAGCTGTATTTGCG 208
1
Met Ala Glu
ACAGTCCATCCATAAAACAAAGATTGACGGAGACAAGGT ATG GCA GAA 256
10
Gln Glu Tyr Asp Leu Ile Val Val Gly Ser Gly Ala Gly
CAA GAA TAT GAC CTG ATC GTC GTG GGT TCG GGG GCA GGT 295
20
Ala Cys Trp Ala Pro Ile Arg Ala Gln Glu Gln Gly Leu
GCC TGT TGG GCG CCA ATT CGC GCG CAG GAG CAG GGC CTC 334
30 40
Lys Thr Leu Val Val Glu Lys Thr Glu Leu Phe Gly Gly
AAG ACT CTG GTG GTG GAG AAG ACC GAG CTG TTC GGT GGC 373
50
Thr Ser Ala Leu Ser Gly Gly Gly Ile Trp Ile Pro Leu
ACT TCG GCT CTG TCG GGG GGT GGT ATC TGG ATT CCG CTC 412
60
Asn Tyr Asp Gln Lys Thr Ala Gly Ile Lys Asp Asp Leu
AAT TAC GAC CAG AAG ACC GCT GGC ATC AAA GAC GAT CTG 451
70 80
Glu Thr Ala Phe Gly Tyr Met Lys Arg Cys Val Arg Gly
GAA ACC GCA TTT GGC TAT ATG AAG CGC TGT GTG CGC GGC 490
90
Met Ala Thr Asp Asp Arg Val Leu Ala Tyr Val Glu Thr
ATG GCA ACC GAC GAC CGC GTG CTG GCC TAT GTG GAA ACC 529
100
Ala Ser Lys Met Ala Glu Tyr Leu Arg Gln Ile Gly Ile
GCG AGC AAG ATG GCC GAG TAC CTG CGC CAG ATC GGC ATC 568
110 120
Pro Tyr Arg Ala Met Ala Lys Tyr Ala Asp Tyr Tyr Pro
CCT TAT CGC GCC ATG GCC AAG TAT GCG GAC TAC TAC CCC 607
130
His Ile Glu Gly Ser Arg Pro Gly Gly Arg Thr Met Asp
CAT ATC GAA GGC TCC AGG CCC GGT GGC CGC ACC ATG GAC 646

140

Pro Val Asp Phe Asn Ala Ala Arg Leu Arg Val Thr Ala
CCG GTG GAC TTC AAT GCC GCC AGG CTG CGC GTG ACG GCA 685
150
Leu Glu Thr Met Arg Pro Gly Pro Pro Gly Asn Gln Leu
CTG GAA ACC ATG CGC CCC GGC CCT CCC GGC AAC CAG TTG 724
160 170
Phe Gly Arg Met Ser Ile Ser Ala Phe Glu Ala His Ser
TTC GGC CGC ATG AGC ATC AGT GCC TTC GAG GCG CAT TCC 763
180
Met Leu Ser Arg Glu Leu Lys Ser Arg Phe Thr Ile Leu
ATG CTC TCG CGC GAG CTC AAG TCG CGC TTC ACC ATC CTG 802
190
Gly Ile Met Leu Lys Tyr Phe Leu Asp Tyr Pro Trp Arg
GGC ATC ATG CTC AAG TAT TTT CTG GAC TAC CCC TGG CGC 841
200 210
Asn Lys Thr Arg Arg Asp Arg Arg Met Thr Gly Gly Gln
AAC AAG ACC AGG CGC GAT CGT CGC ATG ACG GGC GGC CAG 880
220
Ala Leu Val Ala Gly Leu Leu Thr Ala Ala Asn Lys Ala
GCG CTG GTG GCA GGC CTG CTG ACT GCT GCC AAC AAG GCC 919
230
Arg Val Glu Met Trp Cys Asn Ser Pro Leu Lys Glu Leu
CGC GTC GAG ATG TGG TGC AAC TCT CCG CTC AAG GAG CTG 958
240 250
Val Gln Asp Ala Ser Gly Arg Val Thr Gly Val Ile Val
GTG CAG GAT GCA TCG GGC CGC GTG ACG GGT GTC ATT GTT 997
260
Glu Arg Asn Gly Gln Arg Gln Gln Ile Asn Ala Arg Arg
GAG AGG AAT GGT CAG CGC CAG CAG ATC AAC GCC AGA CGT 1036
270
Gly Val Leu Leu Gly Ala Gly Gly Phe Glu Arg Asn Gln
GGC GTG TTG TTG GGT GCA GGC GGT TTT GAG CGC AAT CAG 1075
280
Glu Met Arg Asp Gln Tyr Leu Asn Lys Pro Thr Arg Leu
GAG ATG CGT GAC CAG TAT CTG AAC AAG CCC ACA AGG CTG 1114
290 300
Val Asp Gly Asn Pro Cys Gly Arg Gln Tyr Gly Asp Ala
GTG GAC GGC AAC CCC TGT GGG AGG CAA TAC GGT GAT GCT 1153

310
His Arg Ala Gly Gln Ala Trp Ala His Thr Gly Ala Asp
CAC CGG GCC GGT CAG GCG TGG GCG CAC ACT GGC GCT GAT 1192
320
Gly Leu Val Leu Gly Arg Ala His His Gly Cys Ser Gln
GGA CTG GTC CTG GGG CGT GCC CAC CAT GGA TGT TCC CAA 1231
330 340
Gly Ala Gly Leu Ser Arg His Phe Arg Gly Thr Leu Ala
GGA GCC GGC CTT TCG CGG CAT TTT CGT GGA ACG CTC GCT 1270
350
Ala Gly Val His Gly Gly Gln Arg Gln Gly Ala Ala Leu
GCC GGG GTG CAT GGT GGT CAA CGA CAA GGG GCA GCG CTT 1309
360
Pro Gln Arg Val Arg Pro Val Ser Gly Ile Pro Ala Ala
CCT CAA CGA GTC CGG CCC GTA TCC GGA ATT CCA GCA GCC 1348
370 380
Met Leu Ala Glu Asn Ala Lys Gly Asn Gly Gly Val Pro
ATG CTG GCC GAA AAT GCC AAG GGC AAT GGC GGT GTG CCT 1387
390
Ala Trp Ile Val Phe Asp Ala Ser Phe Arg Ala Gln Asp
GCA TGG ATT GTG TTC GAC GCC AGC TTC CGC GCG CAA AAC 1426
400
Pro Met Gly Pro Leu Met Pro Gly Ser Ala Val Pro Asp
CCC ATG GGG CCG CTG ATG CCA GGC TCG GCC GTG CCA GAC 1465
410
Ser Lys Val Arg Lys Ser Trp Leu Asn Asn Val Tyr Trp
AGC AAG GTG CGC AAG AGC TGG CTG AAC AAT GTC TAC TGG 1504
420 430
Lys Gly Arg Arg Trp Lys Ile Trp Arg Ala Asp Arg Arg
AAG GGC AGA CGC TGG AAG ATC TGG CGC GCA GAT CGG CGT 1543
440
Gly Arg Ala Gly Leu Gln Val Ser Ala Arg Arg Met Thr
GGA CGT GCT GGG CTG CAG GTC AGT GCG CGT CGC ATG ACC 1582
450
Glu Tyr Ala Arg Ala Gly Lys Asp Leu Asp Phe Asp Arg
GAA TAC GCC AGA GCT GGC AAG GAC CTG GAC TTT GAC CGG 1621

460                                                    470
Gly Gly Asn Val Phe Asp Arg Tyr Tyr Gly Asp Pro Arg
GGC GGC AAT GTG TTT GAC CGC TAC TAC GGC GAT CCG CGT   1660
                                        480
Leu Lys Asn Pro Asn Leu Gly Pro Ile Glu Lys Gly Pro
CTC AAG AAT CCC AAC CTG GGT CCC ATC GAG AAA GGT CCG   1699
                        490
Phe Tyr Ala Met Arg Leu Trp Pro Gly Glu Ile Gly Thr
TTC TAC GCC ATG CGT CTG TGG CCC GGT GAG ATC GGC ACC   1738
        500                                     510
Lys Gly Gly Leu Leu Thr Asp Arg Glu Gly Arg Val Leu
AAG GGG GGG CTG CTG ACC GAC AGG GAA GGC CGT GTG CTC   1777
                                            520
Asp Thr Gln Gly Arg Ile Ile Glu Gly Leu Tyr Cys Val
GAC ACG CAA GGC AGG ATC ATC GAG GGG CTG TAT TGC GTG   1816
                            530
Gly Asn Asn Ser Ala Ser Val Met Ala Pro Ala Tyr Ala
GGC AAC AAC TCC GCC TCC GTC ATG GCG CCG GCC TAC GCC   1855
                540
Gly Ala Gly Ser Thr Leu Gly Pro Ala Met Thr Phe Ala
GGC GCT GGC TCC ACC CTG GGG CCG GCC ATG ACG TTT GCC   1894
550                                         560
Phe Arg Ala Val Ala Asp Met Val Gly Lys Pro Leu Pro
TTC CGC GCC GTG GCT GAC ATG GTA GGC AAA CCC TTG CCT   1933
                                    570         573
Leu Glu Asn Pro His Leu Leu Gly Lys Thr Val
CTC GAG AAC CCG CAT CTG CTG GGC AAG ACG GTT TGACCAGG   1974
AGGCGCAGAGATGTCCAATGTCAAAAGCATGTCAGCACCATCAATCCTGTG   2026
GGGGAAGTCCTGGATGTGGGGTCGGCCGATGAGGTTCAGTGGTCCGATGCCA   2078
GCGATGTGGTGGTGGTCGGCTGGGAGGGGCAGGTGCCAGTGCAGCGATTGA   2130
GGCGCGCGAGCAGGGGGCAGAAGTTCTGGTGATCGAGCGCTTCAGCGGTGGC   2182
GGTGCCAGTGTGCTGTCGGGCGGTGTGGTCTATGCCGGTGGCGGTACC   2230

3. Nucleic acid sequence according to any one of claims 1 or 2, characterized in that the sequence coding for delta¹-dehydrogenase of P. testosteroni starts at nucleotide 248 and finishes at nucleotide 1966 of the sequence (I) SEQ ID No. 1.

4. Recombinant vectors, characterized in that they contain a nucleic acid sequence according to any one of claims 1 to 3.

5. Recombinant vector according to claim 4, characterized in that it is constituted by the plasmid called pTEK21, which plasmid results from the insertion in plasmid pUC18 of a DNA sequence having the sequence SEQ ID No. 1.

6. Recombinant vector according to claim 4, characterized in that it is constituted by the recombinant plasmid called pLE689, which plasmid results from the insertion in plasmid pNM185 of the nucleotide sequence coding for delta¹-dehydrogenase of P. testosteroni combined with other nucleotide sequences originating from the genome of P. testosteroni.

7. Clones of transformed microorganisms, characterized in that they contain at least one recombinant vector according to any one of claims 4 to 6.

8. Clone according to claim 7, deposited at the Collection Nationale de Cultures de Micro-organismes (CNCM) under the number I-922, and constituted by bacterial cells of the strain E. coli DH5alpha, containing plasmid pTEK21.

9. Clone according to claim 7, deposited at the Collection Nationale de Cultures de Micro-organismes (CNCM) under the number I-923, and constituted by bacterial cells of the strain E. coli RR1, containing plasmid pLE689.

10. Clone according to claim 7, deposited at the Collection Nationale de Cultures de Micro-organismes (CNCM) under the number I-924, and constituted by bacterial cells of the strain P. putida KT2440, containing plasmid pLE689.

11. Clone according to claim 7, deposited at the Collection Nationale de Cultures de Micro-organismes (CNCM) under the number I-925, and constituted by bacterial cells of the strain P. aeruginosa PA01161, containing plasmid pLE689.

12. Use of microorganisms transformed by a recombinant vector according to claim 4, for the bioconversion of steroids.

13. Use of microorganisms transformed by a recombinant vector according to claim 4, for the production of delta¹-dehydrogenase.

14. Detection probes characterized in that they contain at least one nucleic acid sequence according to any one of claims 1 or 2, combined with at least one appropriate means of detection.

15. Polypeptide chain containing an amino acid sequence resulting from the translation of the DNA sequence according to claim 3.

16. Preparation process for delta¹-dehydrogenase of Pseudomonas testosteroni, characterized in that it includes the following stages:
a) culture of a microorganism containing a vector according to claim 4 allowing the expression of the gene of delta¹-dehydrogenase of Pseudomonas testosteroni,
b) isolation, from the culture, of the delta¹-dehydrogenase produced by said microorganism.

17. Use of delta¹-dehydrogenase of Pseudomonas testosteroni, purified by the process according to claim 16, for the synthesis of steroids.

18. Use of delta¹-dehydrogenase of Pseudomonas testosteroni, purified by the process according to claim 16, for the assay of steroids.
